# EUROPÄISCHE PATENTANMELDUNG

(11) **EP 2 604 233 A2**
(43) Veröffentlichungstag der Anmeldung: **19.06.2013**
(21) Anmeldenummer: 12190720.8
(22) Anmeldetag: 31.10.2012
(51) Int. Cl.: A61F 2/97

(54) **Freigabevorrichtung zum Lösen eines medizinischen Implantats von einen Katheter und Katheter mit einer Freigabevorrichtung sowie Verfahren zum Herstellen eines Klemmkörpers einer Freigabevorrichtung zum Lösen eines medizinischen Implantats von einen Katheter und Katheter mit einer Freigabevorrichtung sowie Verfahren zum Herstellen eines Klemmkörpers einer Freigabevorrichtung und Verfahren zum Klemmen eines Imp-lantats in einem solchen**

(30) Priorität: 14.12.2011 US 201161570312 P
(71) Anmelder: Biotronik AG, 8180 Bülach (CH)
(72) Erfinder: Fargahi, Amir, 8180 Bülach (CH)
(74) Vertreter: Lindner-Vogt, Karin L.

(57) **Zusammenfassung**

Die Erfindung betrifft eine Freigabevorrichtung (100) zum Lösen eines medizinischen Implantats (105) von einer Einführvorrichtung (110), bei welcher das Implantat (105) durch eine Relativbewegung zwischen einem ersten und einem zweiten Einführelement (52, 54) freisetzbar ist, umfassend einen Klemmkörper (10) zum Klemmen des Implantats (105) in der Einführvorrichtung (110), mit einem proximalen Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, wobei der Klemmkörper (10) das Implantat (105) im Klemmzustand an zumindest einer Stelle (106) kappenartig umschließt.

Die Erfindung betrifft ferner eine Einführvorrichtung (110) mit einer solchen Freigabevorrichtung (100) sowie ein Verfahren zum Herstellen eines Klemmkörpers (10) einer Freigabevorrichtung (100) und ein Verfahren zum Klemmen eines Implantats (105) in einem Klemmkörper (10).

## Beschreibung

Die Erfindung betrifft eine Freigabevorrichtung zum Lösen eines medizinischen Implantats von einem Katheter und einen Katheter mit einer Freigabevorrichtung zum Freigeben eines medizinischen Implantats zur Implantation in einen tierischen und/oder menschlichen Körper sowie ein Verfahren zum Herstellen eines Klemmkörpers einer Freigabevorrichtung und Verfahren zum Klemmen eines Implantats in einem solchen Klemmkörper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate werden vor einem Einführen in den Körper mit Kathetern verbunden und müssen so befestigt sein, dass sie am Einsatzort komplikationslos vom Katheter genau platziert und definiert freigegeben werden können. Hierzu ist beispielsweise bekannt, das Implantat mit Ösen auszustatten, die mit Haken am Katheter interagieren und so das Implantat am Katheter befestigen.

Der Erfindung liegt die Aufgabe zugrunde, eine Freigabevorrichtung anzugeben, mit der ein Verbinden eines Implantats mit einer Einführvorrichtung einfach und bedienerfreundlich möglich ist und mit der ein hochpräzises und gezieltes Freigeben eines Implantats erfolgen kann.

Eine weitere Aufgabe ist in der Bereitstellung einer entsprechenden Einführvorrichtung zu sehen.

Ferner besteht eine weitere Aufgabe darin, ein Verfahren zum Herstellen eines Klemmkörpers einer solchen Freigabevorrichtung bereit zu stellen.

Zudem ergibt sich eine weitere Aufgabe in der Bereitstellung eines Verfahrens zum Klemmen eines Implantats mittels eines solchen Klemmkörpers der entsprechenden Freigabevorrichtung.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst einen Klemmkörper zum Klemmen des Implantats in der Einführvorrichtung mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei der Klemmkörper das Implantat im Klemmzustand an zumindest einer Stelle kappenartig umschließt.

Durch die erfindungsgemäße Ausgestaltung kann eine Freigabevorrichtung bereitgestellt werden, in der das Implantat sicher gehalten wird bzw. auf einem Einführelement, wie beispielsweise einem Innenschaft, der Einführvorrichtung sicher befestigt ist. Der Klemmkörper erlaubt ferner eine kompakte und einfache Konstruktion der Freigabevorrichtung. Zudem weist die Freigabevorrichtung eine einfache Handhabung und Montage des Implantats auf der Einführvorrichtung, beispielsweise einem Katheter, im Vorbereitungslabor auf. Des Weiteren ist eine Freigabe des Implantats zuverlässig und schnell. Ferner kann ein Risiko einer Deformation des Implantats sowie einer daraus resultierende Blockade der Freigabevorrichtung, wie sie bei Vorrichtungen des Standes der Technik, die mit Haken und Ösen arbeiten, auftreten, eliminiert werden.

In diesem Zusammenhang soll unter einem "Klemmkörper" ein Körper verstanden werden, der mittels einer Klemmwirkung und/oder eines Kraftschlusses ein anderes Element, insbesondere das Implantat, an einem Bauteil der Einführvorrichtung, insbesondere einem Einführelement bzw. dem Innenschaft, in einer festgelegten Position hält. Hierbei kann der Klemmkörper die Klemmwirkung selbst vermitteln oder er kann mit zumindest einem weiteren Element, insbesondere der Einführvorrichtung, wie beispielsweise einem Einführelement bzw. dem Innenschaft und/oder einem Außenschaft der Einführvorrichtung, zusammenwirken. Zusätzlich kann der Klemmkörper eine von der Klemmwirkung separat ausgelegte Haltewirkung, beispielsweise durch eine Materialeigenschaft, aufweisen. Ein "Klemmzustand" stellt hier einen Zustand dar in dem das Implantat verliersicher in der Einführvorrichtung gehalten wird. Unter "kappenartig" soll hier verstanden werden, dass der Klemmkörper die zumindest eine Stelle des Implantats in Umfangsrichtung des Implantats und in zumindest einem Bereich, der sich entlang einer Ebene erstreckt, die senkrecht zu einer axialen Richtung des Implantats angeordnet ist, umschließt.

Des Weiteren wird vorgeschlagen, dass der Klemmkörper zumindest zwei Hüllteile aufweist, wodurch der Klappenkörper vorteilhaft an eine eventuell variable Geometrie des Implantats angepasst werden kann und/oder ist. Unter einem "Hüllteil" soll hier ein Anteil des Klappenkörpers verstanden werden, der sich im Klemmzustand zumindest über einen Bereich eines Umfangs des Implantats erstreckt und/oder dieses zumindest teilweise umschließt. Die Hüllteile sind bevorzugt an demselben Ende des Klemmkörpers angeordnet, vorteilhafterweise im montierten Zustand des Klemmkörpers in der Einführvorrichtung am distalen Ende. Des Weiteren kann ein Ende des Klemmkörpers relativ zum anderen Ende in seiner Erstreckung und/oder in seinem Durchmesser verbreitert und/oder aufgeweitet sein. Dies kann mit jeder, dem Fachmann für anwendbar erachteten Methode erfolgen und/oder ist insbesondere abhängig von einem Material des Klemmkörpers. Bevorzugt wird das Ende mittels Hitzezufuhr aufgeweitet. Bevorzugt ist das distale Ende aufgeweitet, wodurch auch die Hüllteile bzw. deren distalen Enden aufgeweitet sind. Grundsätzlich könnte der Klemmkörper auch elastisch ausgeführt sein und das Ende könnte im Klemmzustand elastisch verbreitert sein.

Durch die aufgeweitete Ausführung der Enden der Hüllteile können sich diese bei einer Zusammenführung ihrer Enden an den Enden in einer Richtung ihrer Erstreckung und/oder in Umfangsrichtung überlappen, wodurch ein Klemmen des Implantats unterstützt wird. Dies ist insbesondere der Fall, wenn die überlappenden Bereiche der Enden ein Material mit hoher Haftreibung aufweisen. Durch das teilweise Überlappen der Enden der Hüllteile kann eine ursprüngliche Erstreckung und/oder ein ursprünglicher Durchmesser des aufgeweiteten Endes, die bzw. der vor dessen Aufweitung vorlag und die bzw. der einer Erstreckung bzw. einem Durchmesser des gegenüberliegenden Ende entspricht, konstruktiv einfach eingestellt werden. Hierdurch wird ein Klemmkörper bereitgestellt, der zum einen eine hohe Klemmwirkung vermittelt und zum anderen eine homogene Kontur in axialer Richtung aufweist. Dies erlaubt zudem eine kompakte Bauweise der Einführvorrichtung.

In einer vorteilhaften Ausgestaltung wird vorgeschlagen, dass im Klemmzustand die zwei aufgeweiteten sich zumindest teilweise überlappenden Hüllteile des Klemmkörpers zumindest ein proximales Ende des Implantats umschließen, wodurch der Klemmkörper hin zum Anwender bzw. zum proximalen Ende der Einführvorrichtung angeordnet ist und dadurch beispielsweise zusätzlich eine Angriffsfläche für eine Schiebebewegung des Implantats in der Einführvorrichtung bietet. Des Weiteren kann auch ein proximales Ende des Klemmkörpers einen Stopper aufweisen, der zur Limitierung einer Bewegung des Implantats in Richtung eines proximalen Endes der Einführvorrichtung, die zu einer Blockade des Implantats führen würde, dient.

Vorteilhafterweise hält im Klemmzustand eine Wechselwirkung zwischen einer Haltekraft eines Einführelements und einer Haltekraft des Klemmkörpers das Implantat in Position, wodurch ein Entgleiten des Implantats aus der Freigabevorrichtung bzw. dem Klemmkörper verhindert wird. Ferner ist es vorteilhaft, wenn der Klemmkörper aufgrund einer selbst aufgewendeten Kraft öffenbar ist. Durch die selbsttätige Öffnung kann eine hohe Präzision bei der Positionierung des Implantats erreicht werden, statt wie im Stand der Technik Haken und Ösen einzusetzen. Gemäß einer bevorzugten Ausgestaltung, ist der Klemmkörper aufgrund einer Radialkraft des Implantats öffenbar, wodurch die Freigabevorrichtung Bauteil, Bauraum, Montageaufwand, und Kosten sparend realisiert werden kann, da auf ein separates Freigabemittel verzichtet werden kann. Zudem ermöglicht dies eine leichte und kontrollierte Bedienbarkeit der Freigabevorrichtung.

In einer weiteren Ausgestaltung der Erfindung wird vorgeschlagen, dass der Klemmkörper eine Durchführung für eines der Einführelemente aufweist. Dies erlaubt eine kompakte Anordnung, welche das durchgeführte Einführelement stabilisiert und schützt. Ist die Einführvorrichtung ein Katheter, so kann das betreffende Einführelement ein Innenschaft des Katheters sein.

Ferner wird vorgeschlagen, dass der Klemmkörper ein Material mit hoher Haftreibung aufweist, um das Implantat in dem Klemmzustand in Position zu halten, wodurch eine Fixierung des Implantats konstruktiv einfach erfolgen kann. Hierbei wird die Haftreibung zwischen dem Klemmkörper und dem proximalen Ende des Implantats in der Einführvorrichtung erzeugt. Das Material kann jedes, vom Fachmann für sinnvoll erachtetes Material sein, wie insbesondere ein Polymer und insbesondere ein Material ausgewählt aus der Gruppe bestehend aus Polyamid, Polyester, Polyether-Block-Amid, Silikon, Polyurethan. Hierdurch kann der Klemmkörper insbesondere mit geringem Gewicht ausgeführt werden. Eine besonders zuverlässige Positionierung des Implantats in der Einführvorrichtung kann wegen seiner hohen Haftreibung vorteilhaft erreicht werden, wenn das Material z.B. ein Polyether-Block-Amid wie PEBAX der Firma Arkema ist. Hierbei sind alle Härtegrade einsetzbar. In einer bevorzugten Realisierung ist der Klemmkörper mit einem Monolayer-Design ausgeführt. Hierfür können insbesondere Materialien wie Polyamide, Polyester oder Polyether-Block-Amide, wie bevorzugt PEBAX eingesetzt werden.

In einer alternativen Ausgestaltung ist der Klemmkörper mit einem Multilayer-Design bzw. mit einem so genannten Multilayer-Co-extrudiertes Design ausgeführt. Hierbei weist der Klemmkörper zumindest zwei Schichten auf, die in radialer Richtung hintereinander angeordnet sind. Die radial äußere Schicht bzw. die Außenschicht ist hierbei aus einem Material mit geringer Reibung ausgewählt und die radial innere Schicht bzw. die Innenschicht aus einem Material mit höherer Reibung. Die Außenschicht weist hierbei bevorzugt ein Material ausgewählt aus der Gruppe bestehend aus Perfluor-Ethylen-Propylen (FEP), high density Polyethylen (HDPE), Polytetrafluorethylen (PTFE, Teflon) oder Polyamid (PA), insbesondere PA-6, - 6.6, -6.10, -6.12, -11, -12, auf. Die Innenschicht weist hierbei bevorzugt ein Material ausgewählt aus der Gruppe bestehend aus Polyamid, Polyester, Polyether-Block-Amid (PEBAX), Silikon, Polyurethan, (PUR) auf.

Zu einer guten Kontaktierung der zwei Schichten kann eine weitere, mittlere Schicht vorgesehen sein. Diese mittlere Schicht ist bevorzugt aus einem Material gefertigt, das als ein Haftvermittler geeignet ist, d.h. eine Haftung zwischen Außen- und Innenschicht erzeugen kann. Die mittlere Schicht weist bevorzugt das Material linear low density Polyethylen (LLDP) auf. Des Weiteren kann auf der radial äußeren Schicht bzw. der Außenschicht eine Beschichtung zur Reduktion der Reibung eingesetzt werden. Diese Beschichtung kann hydrophob oder hydrophil sein und aus jedem, dem Fachmann für sinnvoll erachtetem Material bestehen.

Gemäß einem weiteren Aspekt der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine Freigabevorrichtung zum Lösen des medizinisches Implantats, aufweisend einen Klemmkörper zum Klemmen des Implantats in der Einführvorrichtung mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei der Klemmkörper das Implantat im Klemmzustand an zumindest einer Stelle kappenartig umschließt.

Durch die erfindungsgemäße Ausgestaltung kann eine Einführvorrichtung bereitgestellt werden, in der das Implantat sicher gehalten wird bzw. auf dem Innenschaft der Einführvorrichtung sicher befestigt ist. Der Klemmkörper erlaubt ferner eine kompakte und einfache Konstruktion der Einführvorrichtung. Zudem kann das Implantat einfach auf der Einführvorrichtung bzw. dem Katheter montiert werden. Des Weiteren ist die Freigabe des Implantats zuverlässig und schnell. Ferner kann ein Risiko einer Deformation des Implantats sowie einer daraus resultierende Blockade der Einführvorrichtung, wie sie bei Vorrichtungen des Standes der Technik, die mit Haken und Ösen arbeiten, auftreten, eliminiert werden. Die Einführvorrichtung kann günstigerweise ein Katheter sein. Besonders vorteilhaft kann die Einführvorrichtung zur Montage und Freigabe einer Prothese, einer Herzklappe oder eines Stents eingesetzt werden.

Zudem wird vorgeschlagen, dass die Einführvorrichtung einen Stopper aufweist, der eine Bewegung des Implantats in Richtung eines proximalen Endes der Einführvorrichtung limitiert. Solch eine Bewegung würde unvorteilhaft zu einer Blockade des Implantates führen. Eine bevorzugte Weiterbildung besteht darin, dass der Klemmkörper an dem Stopper anliegt, wodurch eine Position des Klemmkörpers während der Bewegung des äußeren Einführelements in Richtung eines proximalen Endes der Einführvorrichtung bzw. der Relativbewegung der beiden Einführelemente klar festgelegt ist. Des Weiteren kann der Stopper mit dem Klemmkörper verbunden sein, wodurch ein Verrücken des Klemmkörpers relativ zum Stopper effektiv verhindert werden kann. Hierbei kann jede, dem Fachmann für sinnvoll erachtete Verbindungsart, wie ein Kraft-, ein Form- oder ein Stoffschluss, in Frage kommen. Besonders bevorzugt ist der Klemmkörper mit dem Stopper einstückig ausgeführt, wodurch die Anordnung sehr stabil ausgeführt sein kann. Hierbei soll unter "einstückig" verstanden werden, dass der Stopper und der Klemmkörper von demselben Bauteil gebildet sind und/oder nur unter Funktionsverlust zumindest eines der Bauteile voneinander getrennt werden können.

Gemäß einer vorteilhaften Ausgestaltung kann das Implantat ein selbstexpandierendes Implantat sein, wodurch es selbsttätig durch seine Radialkraft den Klemmkörper öffnen kann. Durch das selbstexpandierende Implantat kann ein zusätzliches Expandiermittel entfallen. Dadurch können vorteilhaft Raum und Montageaufwand für dieses eingespart werden. Hierdurch kann auch die Einführvorrichtung weniger komplex gestaltet werden. Grundsätzlich wäre es jedoch auch möglich ein ballonexpandierbares Implantat zu verwenden. Hierfür müsste die Einführvorrichtung jedoch entsprechend angepasst werden, was der Fachmann aufgrund seiner Fachkenntnis selbstständig löst. Besonders vorteilhaft ist das Implantat befestigungselementlos ausgebildet, wodurch das Implantat gegenüber Implantaten des Standes der Technik verkürzt werden kann. Dies wirkt sich vor allem für den Patienten positiv aus. Folglich kann auch die Einführvorrichtung befestigungselementlos ausgestaltet werden. Hierdurch entfällt bei der Montage des Implantats auf der Einführvorrichtung ein sonst anfälliges Verbinden von Befestigungselementen, wie Haken und Ösen, wodurch auch ein Ausschuss wegen Fehlmontagen Kosten sparend reduziert wird. Dies resultiert vor allem in einer Zeitersparnis bei der Vorbereitung der Einführvorrichtung im Vorbereitungslabor.

Es wird ferner ein Verfahren zum Herstellen eines Klemmkörpers einer Freigabevorrichtung zum Klemmen eines medizinischen Implantats in einer Einführvorrichtung, bei welcher das Implantat durch eine Relativbewegung zwischen einem ersten und einem zweiten Einführelement freisetzt wird, vorgeschlagen. Der Klemmkörper umfasst ein proximales Ende, das im Benutzungszustand von einem distalen Ende der Einführvorrichtung entfernt ist, und einem distalen Ende, das im Benutzungszustand dem distalen Ende der Einführvorrichtung zugewandt ist. Das Verfahren, weist zumindest die folgenden Schritte auf: Bereitstellen eines Schlauchs; Aufweiten des Schlauchs an zumindest einem Ende; und Aufspleißen des Schlauchs an einem Ende in wenigstens zwei sich entlang einer Haupterstreckung des Schlauchs erstreckende Hüllteile.

Durch die erfindungsgemäße Ausgestaltung kann ein Verfahren realisiert werden, mittels dem ein Klemmkörper einer Freigabevorrichtung bedienerfreundlich, zuverlässig und schnell hergestellt werden kann. Der Schlauch weist bevorzugt einen im Wesentlichen runden Querschnitt auf und /oder ist aus einem flexiblem Material gebildet. Des Weiteren ist der Schlauch in einer definierten Form bereitgestellt, die an zumindest einen Bereich einer Kontur und/oder an einen Durchmesser des Implantats angepasst ist. Zudem ist eine Außenerstreckung bzw. ein Außendurchmesser des Schlauchs an Dimensionen der Einführvorrichtung wie insbesondere eines Innendurchmessers des Außenschafts angepasst. Unter "Aufspleißen" soll hier eine Einbringung von zumindest einem Schlitz verstanden werden. Dies erfolgt bevorzugt im Wesentlichen parallel zu der Haupterstreckung des Schlauchs vom aufgeweiteten Ende, dem distalen Ende, bis kurz vor das dem aufgeweiteten Ende gegenüberliegenden Ende, dem proximalen Ende. Der Schlauch wird hierbei nicht in zwei separate Teile getrennt, sondern die zumindest zwei Hüllteile bleiben am proximalen Ende über einen Flansch verbunden. Hierbei soll unter "im Wesentlichen parallel" auch eine Abweichung der Richtung des Schlitzes und der Haupterstreckung von 30° verstanden werden. Eine Haupterstreckung des Schlauchs erstreckt sich in axialer Richtung.

In einer bevorzugten Weiterführung wird ein Material des Schlauchs an zumindest einer Stelle reduziert, um eine Stauchung des Materials zu ermöglichen. Hierdurch kann das Umhüllen des Implantats ohne Materialverwerfungen an einer Kontaktstelle der Hüllteile erfolgen. Dies findet bevorzugt zwischen dem Aufweiten und dem Aufspleißen statt. Die Stelle der Materialreduzierung befindet sich bevorzugt an dem dem aufgeweiteten Ende gegenüberliegenden Ende bzw. dem proximalen Ende des Schlauchs. Die Reduzierung erfolgt insbesondere durch die Einbringung einer Öffnung im Wesentlichen senkrecht zur Haupterstreckung des Schlauchs. Hierbei soll unter "im Wesentlichen senkrecht" auch eine Abweichung der Richtung der Öffnung und der Haupterstreckung von 30° verstanden werden. Die Öffnung kann besonders vorteilhaft als Stopppunkt für das Aufspleißen bzw. für den Schlitz verwendet werden.

Zudem wird ein Verfahren zum Klemmen eines medizinischen Implantats mittels eines Klemmkörpers einer Freigabevorrichtung in einer Einführvorrichtung, bei welcher das Implantat durch eine Relativbewegung zwischen einem ersten und einem zweiten Einführelement freisetzt wird, vorgeschlagen. Der Klemmkörper umfasst ein proximales Ende, das im Benutzungszustand von einem distalen Ende der Einführvorrichtung entfernt ist, und einem distalen Ende, das im Benutzungszustand dem distalen Ende der Einführvorrichtung zugewandt ist. Das Verfahren weist zumindest die folgenden Schritte auf: Einführen zumindest eines proximalen Endes des Implantats aus Richtung eines aufgeweiteten Ende in eine Mantelfläche des Klemmkörpers, die von zumindest zwei Hüllteilen des Klemmkörpers gebildet wird; Umschließen des proximalen Endes des Implantats mit der Mantelfläche der zumindest zwei Hüllteile des Klemmkörpers; und Platzierung des Klemmkörpers mit dem Implantat in zumindest einem Einführelement.

Durch die erfindungsgemäße Ausgestaltung kann ein Verfahren realisiert werden, mittels dem ein Implantat bedienerfreundlich, präzise und schnell in der Einführvorrichtung platziert und befestigt werden kann. Wähen des Umschließens des Implantats werden bevorzugt die zumindest zwei aufgeweiteten Enden der Hüllteile ineinander geschoben, sodass diese sich zumindest bereichsweise überlappen. Hierdurch wird vorteilhafterweise eine Form erreicht, die der definierten Form des Schlauchs entspricht, wodurch der Klemmkörper in seiner Klemmposition vorteilhaft an die Kontur des Implantats angepasst ist und selbst eine homogene Kontur aufweist. Ein weiterer vorteilhafter Aspekt des Verfahrens ist darin zu sehen, dass das Implantat während des Umschließens mit der Mantelfläche der zumindest zwei Hüllteile des Klemmkörpers zusammen gedrückt wird, wodurch eine Durchmesserreduzierung des Implantat selbsttätig aufwandsgünstig mittels des Klemmkörpers erfolgen kann.

Die Erfindung ist nachfolgend beispielhaft, anhand von in Zeichnungen dargestellten Ausführungsbeispielen, näher erläutert. Es zeigen in schematischer Darstellung:
- Fig. 1: einen Schnitt durch ein günstiges Ausführungsbeispiel einer Einführvorrichtung und einer Freigabevorrichtung;
- Fig. 2A-E: fünf Stufen der Herstellung eines Klemmkörpers der Freigabevorrichtung aus Fig. 1;
- Fig. 3: der Klemmkörper aus Fig. 2F in einer Draufsicht auf Enden zweier überlappender Hüllteile des Klemmkörpers;
- Fig.4: die Einführvorrichtung mit dem Klemmkörper aus Fig. 2D in einem Zustand vor einer Platzierung eines Implantats;
- Fig. 5: die Einführvorrichtung und der Klemmkörper aus Fig. 4 mit platziertem Implantat;
- Fig. 6: der Klemmkörper und das Implantat aus Fig. 5 jeweils im zusammengedrückten Zustand in einem äußeren Einführelement der Einführvorrichtung, und
- Fig. 7: eine schematische Darstellung von Haltekräften der Einführvorrichtung, des Klemmkörpers und des Implantats.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

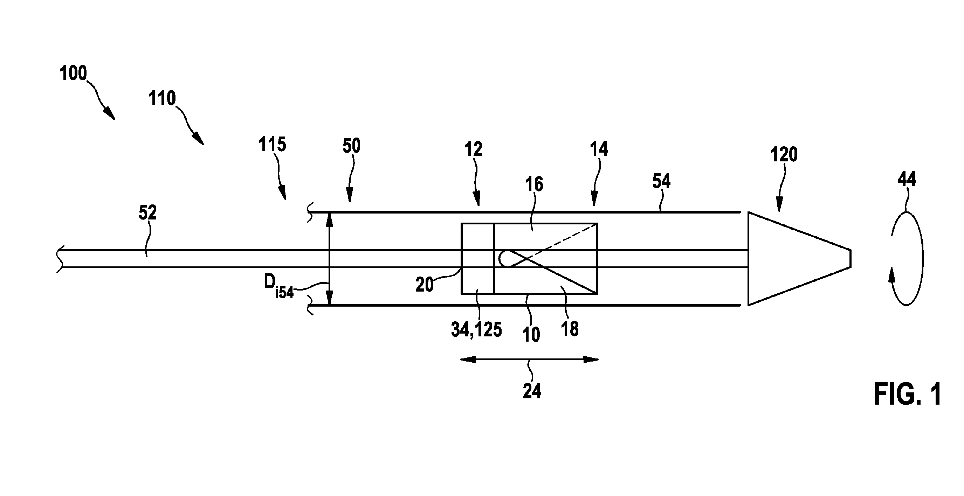

Figur 1 zeigt einen Längsschnitt durch ein günstiges Ausführungsbeispiel einer Freigabevorrichtung 100 einer nur teilweise dargestellten Einführvorrichtung 110. Die Einführvorrichtung 110 ist beispielsweise ein Katheter mit einem Schaftbereich 50 mit zwei koaxial angeordneten Einführelementen 52, 54, z.B. einem Innenschaft (Einführelement 52) und einem diesen umgebenden Außenschaft (Einführelement 54), welcher wiederum von einer nicht gezeigten Außenhülle umgeben sein kann. Die Einführvorrichtung 110 ist in Anwenderbetrieb, also während einer Befestigung des Implantats 105 an der Freigabevorrichtung 100 oder während der Implantation mit ihrem proximalen Ende 115 einem Anwender zugewandt. Das Implantat 105 ist am distalen Ende 120 des Schaftbereichs 50 zwischen Innenschaft und Außenschaft platziert und soll am Implantationsort im tierischen oder menschlichen Körper freigegeben werden.

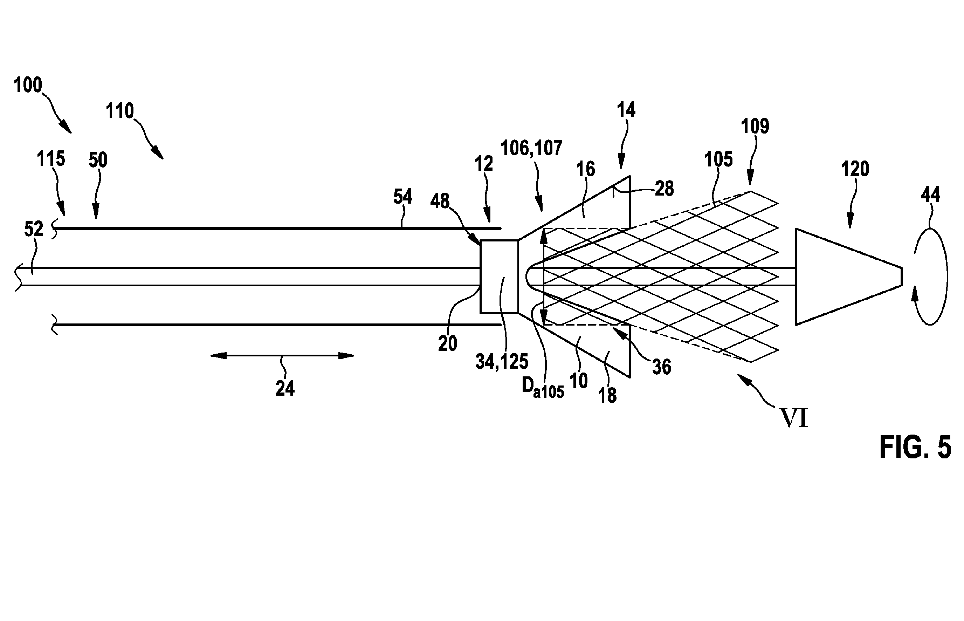

Die Freigabevorrichtung 100 dient zum Lösen des medizinischen Implantats 105 von der Einführvorrichtung 110. Das Implantat 105 ist an einem vom Anwender abgewandten Ende 120 des Schaftbereichs 50 beispielsweise in der Nähe einer Katheterspitze angeordnet (siehe Fig. 5). Das Implantat 105 ist beispielsweise um das innere Einführelement 52 gelegt und wird durch eine Relativbewegung zwischen dem ersten und dem zweiten Einführelement 52, 54 freigesetzt

Die Freigabevorrichtung 100 umfassend einen Klemmkörper 10 zum Klemmen des Implantats 105 in der Einführvorrichtung 110. Zudem weist der Klemmkörper 10 ein proximales Ende 12, das im Benutzungszustand von dem distalen Ende 120 der Einführvorrichtung 110 entfernt ist, und ein distales Ende 14, das im Benutzungszustand dem distalen Ende 120 der Einführvorrichtung 110 zugewandt ist, auf.

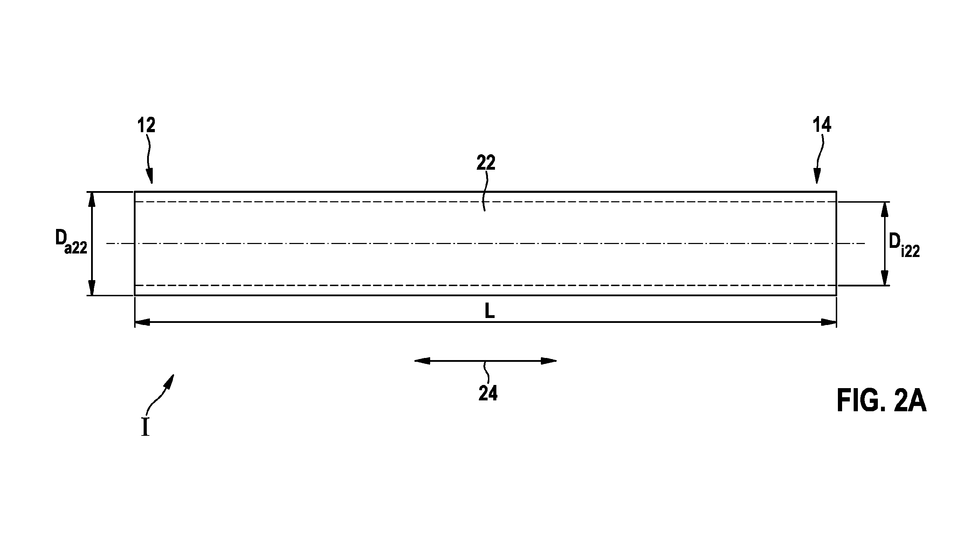

Im Folgenden wird anhand der Fig. 2A bis 2E ein Verfahren zur Herstellung des Klemmkörpers 10 beschrieben. Wie in Fig. 2A zu sehen ist wird ein Schlauchs 22 bereitgestellt (I). Dieser Schlauch 22 ist aus einem Monolayer-Design Polymer (z.B. PEBAX der Firma Arkema) mit hoher Reibung gefertigt, wodurch auch der Klemmkörper 10 aus diesen Materialien gefertigt ist. Alternativ wäre es auch möglich, den Schlauch 22 mit einem so genannten Multilayer-Co-extrudiertem Design mit einer Außenschicht und einer Innenschicht zu fertigen. Hierbei wäre die Außenschicht aus einem Material mit geringer Reibung (z.B. HDPE) und die Innenschicht aus einem Material mit höherer Reibung (z.B. PEBAX) gefertigt. Zudem weist der Schlauch 22 einen im Wesentlichen runden Querschnitt auf. Ferner sind eine Dimensionierung sowie ein Innendurchmesser Dᵢ₂₂ des Schlauchs an eine Kontur oder einen Außendurchmesser Dₐ₁₀₅ des Implantats 105 angepasst (vgl. Fig. 5). Des Weiteren sind diese Parameter des Schlauchs 22 mit Maßen der Einführvorrichtung 110, wie beispielsweise einem Innendurchmesser Dᵢ₅₄ des Einführelements 54 bzw. des Außenschafts, abgestimmt. Hierbei kann beispielsweise ein Außendurchmesser Dₐ₂₂ des Schlauchs 22 der Innendurchmesser Dᵢ₅₄ des Außenschafts subtrahiert um 0,2 mm sein, wodurch das Implantat 105 im montierten Zustand in der Einführvorrichtung 110 eine Radialkraft aufbringen muss, die ausreicht um den Klemmkörper 10 radial um 0,1 mm in Richtung des Außenschafts zu drücken. Ein Innendurchmesser Dᵢ₂₂ beträgt beispielsweise den Außendurchmesser Dₐ₂₂ des Schlauchs 22 subtrahiert um zweimal die Wandstärke des Schlauchs 22, wobei die Wandstärke beispielsweise 0,1 mm - 0,2 mm beträgt. Eine Länge L des Schlauchs 22 kann beispielsweise 15 mm betragen.

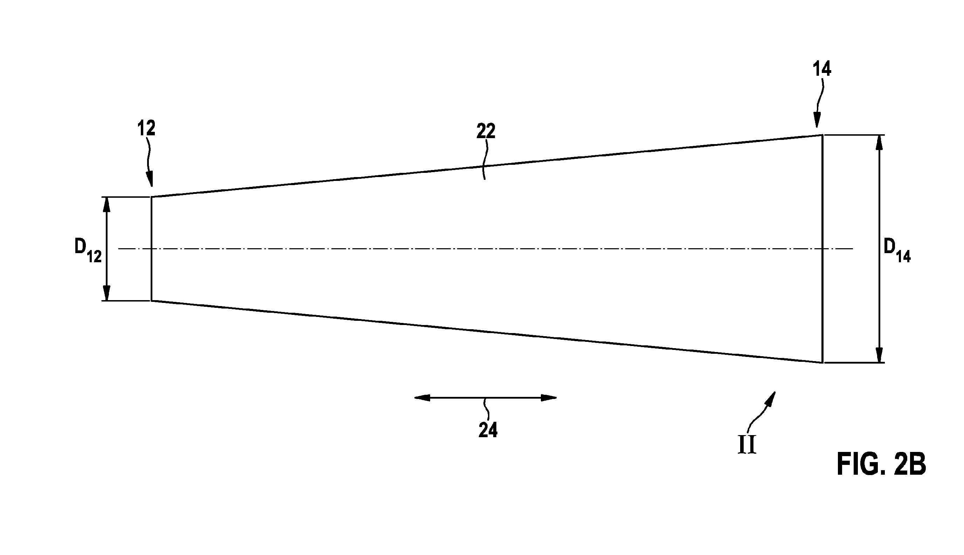

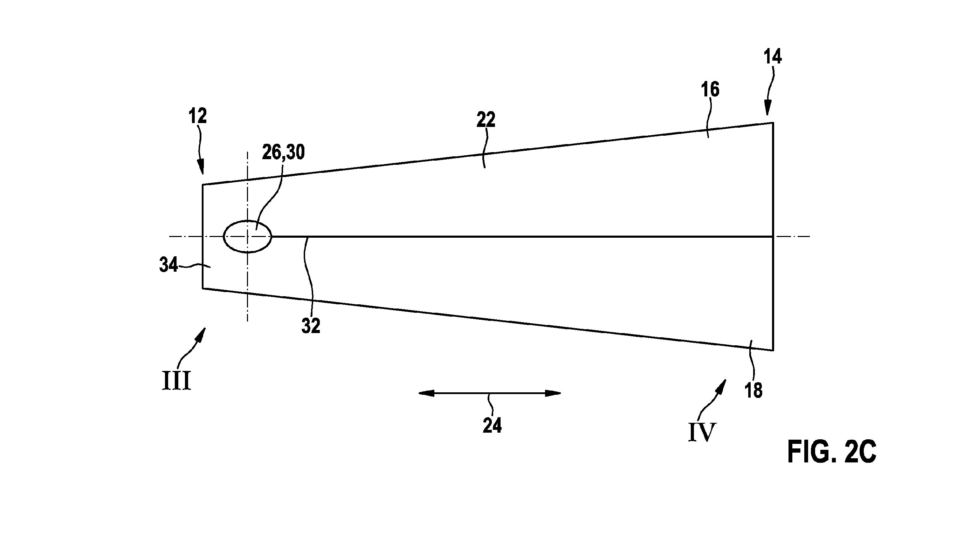

In einem zweiten Schritt wird der Schlauch 22 an einem Ende 14 in seinem Durchmesser D₂₂ zu einem größeren Durchmesser D₁₄ aufgeweitet (II) (Fig. 2B). Dieses Ende 14 stellt im montierten Zustand des Klemmkörpers 10 in der Einführvorrichtung 110 das distale Ende 14 dar. Das Aufweiten kann beispielsweise mittels eines nicht gezeigten Heißdorns oder eines Blasenprozess erfolgen. In einem nachfolgenden dritten Schritt, gezeigt in Fig. 2C, wird das Material des Schlauchs 22 an einer Stelle 26 reduziert (III). Diese Stelle 26 ist an einem dem Ende 14 gegenüberliegenden Ende 12, das im montierten Zustand des Klemmkörpers 10 in der Einführvorrichtung 110 das proximale Ende 12 darstellt, angeordnet. Zudem erfolgt die Materialreduzierung mittels einer Einbringung einer Öffnung 30, die sich senkrecht zu einer Haupterstreckung 24 des Schlauchs 22 in einer axialen Richtung des Schlauchs 22 erstreckt.

In einem vierten, ebenfalls in Fig. 2C gezeigten Schritt, wird der Schlauch 22 an dem proximalen Ende 14 symmetrisch aufgespleißt (IV), wodurch zwei Hüllteile 16, 18 gebildet werden, die sich entlang der Haupterstreckung 24 des Schlauchs 22 erstrecken. Dies erfolgt indem in den Schlauch 22 von seinem aufgeweiteten bzw. distalen Ende 14 parallel zu der Haupterstreckung 24 des Schlauchs 22 bis kurz vor das dem aufgeweiteten Ende 14 gegenüberliegenden proximalen Ende 12 ein Schlitz 32 eingebracht wird. Hierbei dient die Öffnung 30 als Stopppunkt für das Aufspleißen bzw. für den Schlitz 32. Hierdurch sind die Hüllteile 16, 18 am proximalen Ende 12 über einen Flansch 34 verbunden.

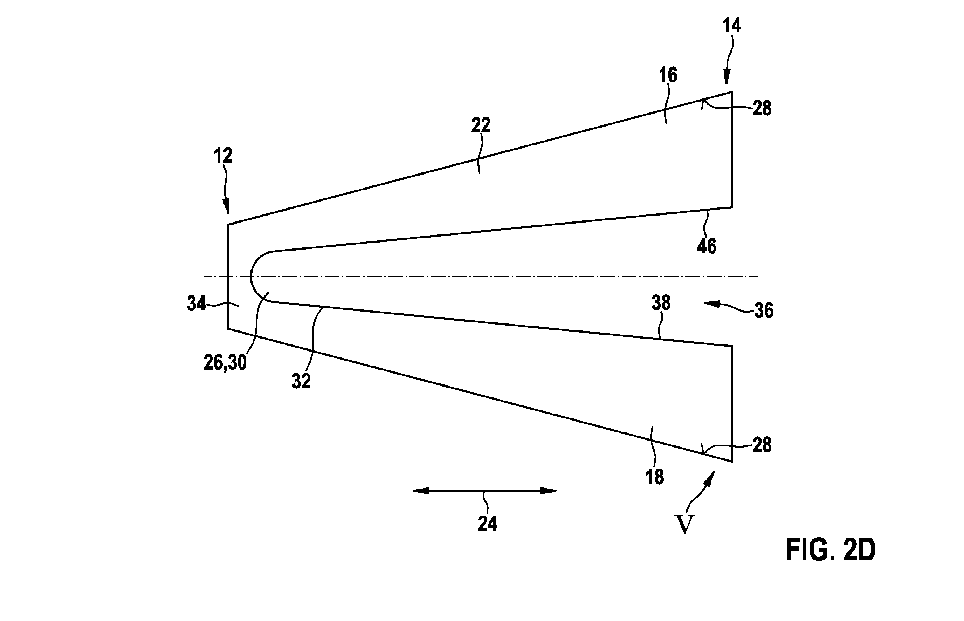

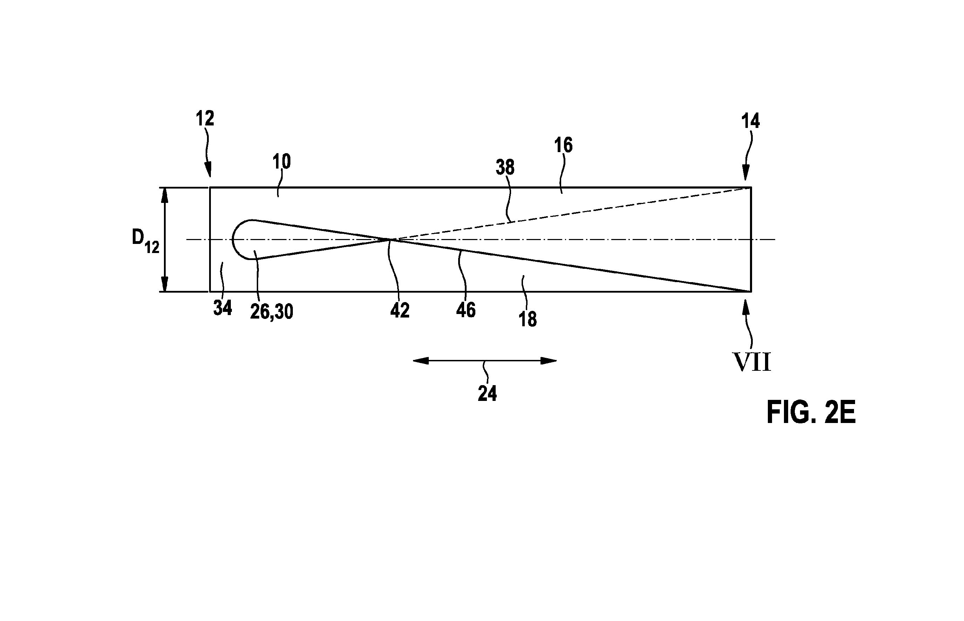

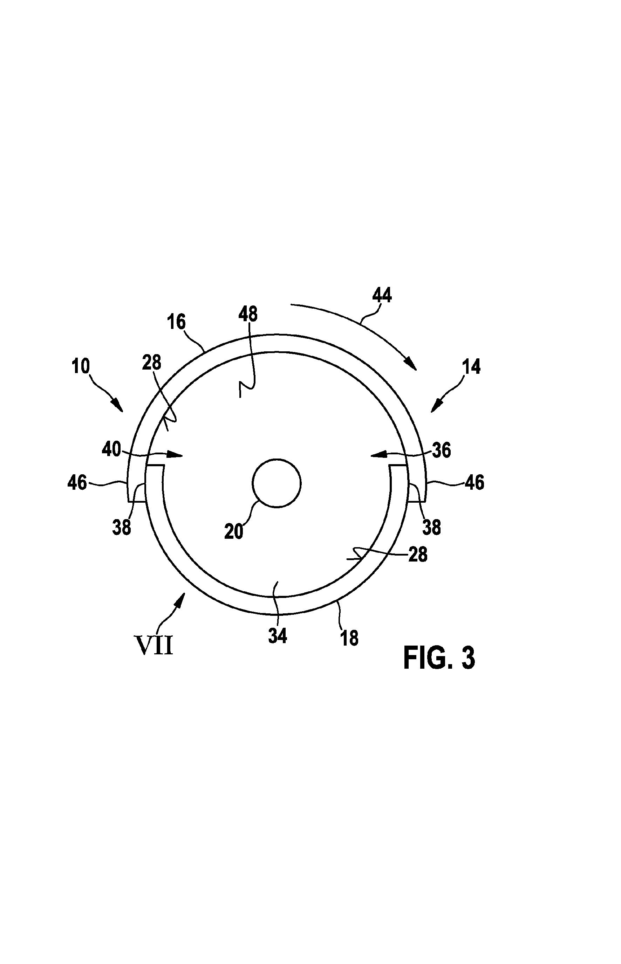

In einem fünften Schritt (siehe Fig. 2D) wird der Schlauch 22 geöffnet (V) und stellt somit eine Aussparung 36, in die das Implantat 105 eingeführt werden kann, bereit. Die Aussparung 36 ist zumindest teilweise von den Hüllteilen 16, 18 umschlossen und weist eine Mantelfläche 28 auf, die somit von den zwei Hüllteilen 16, 18 gebildet wird. Der Klemmkörper 10 ist in dieser offenen Position einsatzbereit. In Fig. 2E ist der Klemmkörper 10 in seiner Klemmposition, exemplarisch ohne Implantat 105 gezeigt. Um diese Position zu erreichen, werden die Hüllteile 16, 18 gegeneinander gestaucht und Kanten 38 des einen Hüllteils 18, die in Richtung des anderen Hüllteils 16 weisen, werden in ein Lumen 40 des anderen Hüllteils 16 geschoben. Dies kann wegen der Öffnung 30 ohne Materialverwerfungen an einer Kontaktstelle 42 der Hüllteile 16, 18 erfolgen, wodurch die Materialreduzierung dazu dient, eine Stauchung des Materials zu ermöglichen (VII). Somit überlappen die Hüllteile 16, 18 in Umfangsrichtung 44 an ihren Kanten 38, 46. Dies ist insbesondere in Fig. 3 zu sehen, die den gestauchten Klemmkörper 10 in einer Draufsicht auf die distalen Enden 14 der überlappenden Hüllteile 16, 18 zeigt. Des Weiteren weist der Klemmkörper 10 in dieser Position am distalen Ende 14 wieder den ursprünglichen Durchmesser D₂₂ des Schlauchs 22 auf, der auch einem Durchmesser D₁₂ des Klemmkörpers 10 am proximalen Ende 12 entspricht. Hierdurch weist der Klemmkörper 10 eine homogene Kontur in seiner Haupterstreckung 24 auf. Ein so gestauchter Klemmkörper 10 kann einfach in das Einführelement 54 bzw. dem Außenschaft eingeführt werden.

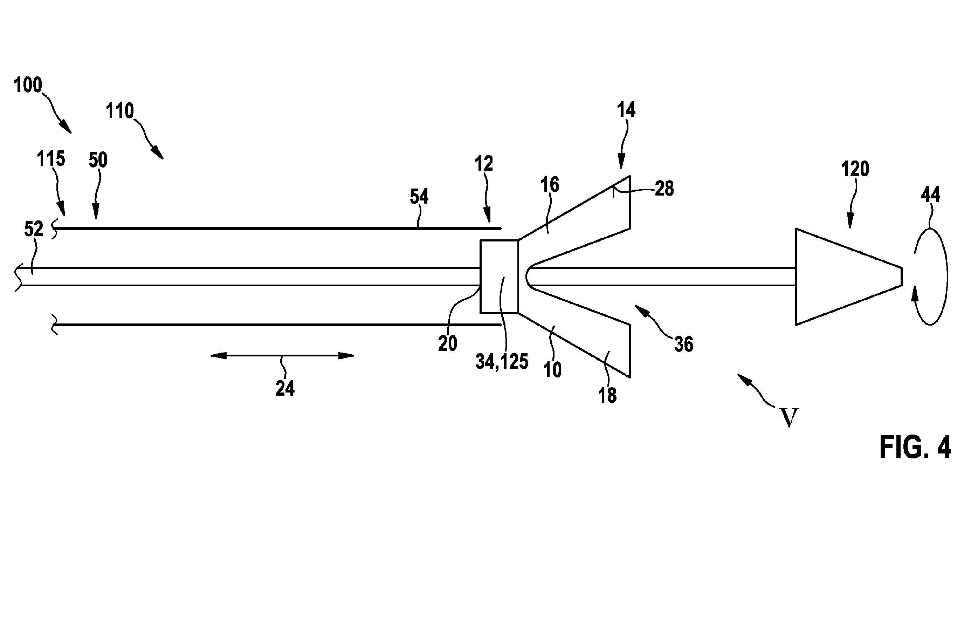

Fig. 1 zeigt den Klemmkörper 10 in solch einer gestauchten Position angeordnet in der Einführvorrichtung 110. Zu einer Anordnung des Klemmkörpers 10 auf dem Einführungselement 52 bzw. dem Innenschaft, weist der Klemmkörper 10 eine Durchführung 20 für das Einführelement 52 auf. In Fig. 4 ist die Einführvorrichtung 110 vor der Platzierung des Implantats 105 gezeigt. Hierfür wurde das äußere Einführelement 54 in Richtung des proximalen Endes 115 der Einführvorrichtung 110 verschoben, bis sich der Klemmkörper 10 durch eine Radialkraft des Implantats 105 selbsttätig öffnet (V). Zu einer Limitierung der Bewegung des Implantats 105 in Richtung des proximalen Endes 115 der Einführvorrichtung 110 weist die Einführvorrichtung 110 einen Stopper 125 auf. Dieser Stopper 125 wird vom Flansch 34 gebildet und ist somit einstückig mit dem Klemmkörper 10 ausgeführt.

In Fig. 5 ist die Platzierung des Implantats 105 im geöffneten Klemmkörper 10 gezeigt. Das Implantat 105, beispielsweise ein Stent oder ein künstliches Herzklappenimplantat, ist selbstexpandierend und befestigungselementlos ausgeführt. Im Folgenden ist ein Verfahren zum Klemmen des Implantats 105 mittels des Klemmkörpers 10 in Fortführung der Verfahrensschritte der Herstellung des Klemmkörpers 10 anhand der Fig. 5 bis 7 beschrieben.

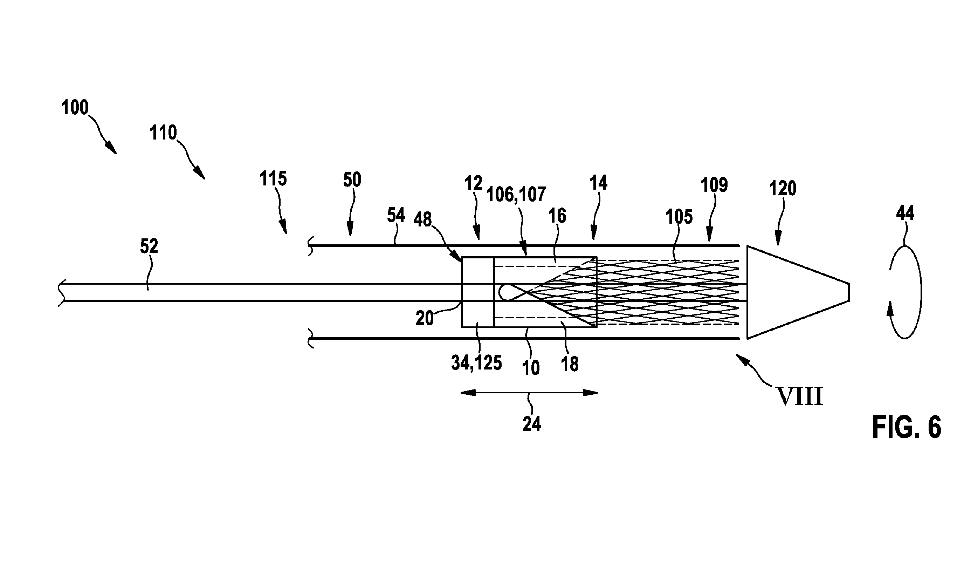

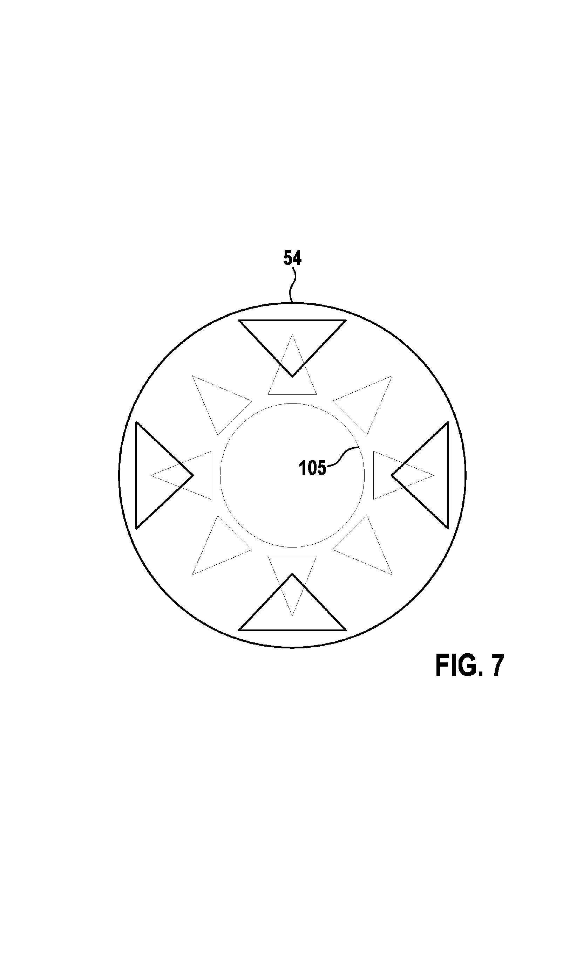

Hierbei wird in einem ersten Schritt ein proximales Ende 107 des Implantats 105, das auf den Innenschaft gecrimpt ist, mit dem Innenschaft in die Mantelfläche 28 der Aussparung 36 des Klemmkörpers 10 eingeführt (VI). Dies erfolgt aus Richtung des aufgeweiteten bzw. distalen Endes 14 des Klemmkörpers 10 (Fig. 5). In einem nachfolgenden, zweiten Schritt werden die Hüllteile 16, 18 gestaucht und somit wird das proximale Ende 107 des Implantats 105 mit der Mantelfläche 28 der zwei Hüllteile 16, 18 umschlossen (VII). Während des Umschließens mit der Mantelfläche 28 der zwei Hüllteile 16, 18 des Klemmkörpers 10 wird das Implantat 105 zusammen gedrückt (VII). Das Umschließen kann manuell oder über ein Verschieben des äußeren Einführelements 54 bzw. den Außenschaft in Richtung des distalen Endes 120 der Einführvorrichtung 110 erfolgen, wodurch der Klemmkörper 10 mit dem Implantat 10 in dem Einführelement 54 platziert wird (VIII) (siehe Fig. 6). Wie in Fig. 6 zudem zu sehen ist, umschließt der Klemmkörper 10 das Implantat 105 im Klemmzustand an einer Stelle 106 bzw. dem proximalen Ende 107 kappenartig. Hierbei sind die Hüllteile 16, 18 in Umfangsrichtung 44 um das proximale Ende 107 des Implantats 105 angeordnet. Zudem ist der Flansch 34 bzw. der Stopper 125 in einer Ebene 48 angeordnet, die sich senkrecht zur Haupterstreckung 24 des Klemmkörpers 10 erstreckt. Somit umschließen im Klemmzustand die zwei aufgeweiteten sich teilweise überlappenden Hüllteile 16, 18 das proximale Ende 107 des Implantats 105. Wie in Fig. 7 dargestellt ist, hält im Klemmzustand eine Wechselwirkung zwischen einer Haltekraft eines Einführelements 54 und Radialkraft des Implantats 105 den Klemmkörper 10 und das Implantat 105 in Position. Zudem weist der Klemmkörper 10 wegen seiner Materialeigenschaften eine hohe Haftreibung auf. Durch die Haftreibung zwischen dem Klemmkörper 10 und dem Implantat 105 weist der Klemmkörper 10 eine Haltekraft auf, die das Implantat 105 in der Einführvorrichtung 110 im Klemmzustand in Position hält, wodurch ein Entgleiten des Implantats 105 verhindert wird. Hierbei ergibt sich für die Haftreibung F_{R} eine Ungleichung: F_{R}≤ µ_{H} • F_{N} mit: µ_{H}: Haftreibungszahl (materialabhängige Zahl), F_{N}: Nominalkraft, resultierend durch die Radialkraft des Implantats 105. Somit hält im Klemmzustand eine Wechselwirkung zwischen der Haltekraft des Einführelements 54 und der Haltekraft des Klemmkörpers 10 das Implantat 105 in Position

Zu einer Implantation des Implantats 105 im Körper, wird die so vorbereitete Einführvorrichtung 110 in den Körper eingeführt (nicht gezeigt). Durch die Bewegung des äußeren Einführelements 54 in Richtung des proximalen Endes 115 der Einführvorrichtung 110 wird bei Freilegung eines distalen Endes 109 des Implantats 105 zuerst dieses wegen seiner Fähigkeit der Selbstexpansion geöffnet und positioniert (nicht gezeigt). Wird der Außenschaft nun bis zum Stopper 125 zurückgezogen, werden die Hüllteile 16, 18 des Klemmkörpers 10 freigelegt, wodurch diese sich aufgrund der Radialkraft des Implantats 105 selbsttätig öffnen (siehe Fig. 5). Hierdurch wird auch das proximale Ende 107 des Implantats 105 aufgrund seiner Radialkraft freigegeben und geöffnet. Im Anschluss wird die Freigabevorrichtung 100 bzw. der Klemmkörper 10 mit dem Innenschaft in den Außenschaft zurückgezogen, wodurch der Klemmkörper 10 wieder gestaucht wird (vgl. Fig. 1) und die Einführvorrichtung 110 aus dem Körper entfernt. Das Implantat 105 verbleibt vollständig positioniert im Körper.

## Patentansprüche

1. Freigabevorrichtung (100) zum Lösen eines medizinischen Implantats (105) von einer Einführvorrichtung (110), bei welcher das Implantat (105) durch eine Relativbewegung zwischen einem ersten und einem zweiten Einführelement (52, 54) freisetzbar ist, umfassend einen Klemmkörper (10) zum Klemmen des Implantats (105) in der Einführvorrichtung (110), mit einem proximalen Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, wobei der Klemmkörper (10) das Implantat (105) im Klemmzustand an zumindest einer Stelle (106) kappenartig umschließt.

2. Freigabevorrichtung nach Anspruch 1, wobei im Klemmzustand zwei aufgeweitete sich zumindest teilweise überlappende Hüllteile (16, 18) des Klemmkörpers (10) zumindest ein proximales Ende (107) des Implantats (105) umschließen.

3. Freigabevorrichtung nach Anspruch 1 oder 2, wobei im Klemmzustand eine Wechselwirkung zwischen einer Haltekraft eines Einführelements (54) und einer Haltekraft des Klemmkörpers (10) das Implantat (105) in Position hält.

4. Freigabevorrichtung nach einem der vorhergehenden Ansprüche, wobei der Klemmkörper (10) aufgrund einer Radialkraft des Implantats (105) öffenbar ist.

5. Freigabevorrichtung nach einem der vorhergehenden Ansprüche, wobei der Klemmkörper (10) eine Durchführung (20) für eines der Einführelemente (52) aufweist.

6. Freigabevorrichtung nach einem der vorhergehenden Ansprüche, wobei der Klemmkörper (10) ein Material mit hoher Haftreibung aufweist, um das Implantat (105) in dem Klemmzustand in Position zu halten.

7. Freigabevorrichtung nach Anspruch 6, wobei das Material ein Polymer und insbesondere ein Material ausgewählt aus der Gruppe bestehend aus Polyamid, Polyester, Polyether-Block-Amid, Silikon, Polyurethan ist.

8. Freigabevorrichtung nach einem der vorhergehenden Ansprüche, wobei der Klemmkörper (10) mit einem Monolayer-Design oder mit einem Multilayer- Design ausgeführt ist.

9. Einführvorrichtung (110) zum Einführen eines medizinischen Implantats (105), welches durch eine Relativbewegung zwischen einem ersten und einem zweiten Einführelement (52, 54) freisetzbar ist, umfassend eine Freigabevorrichtung (100) zum Lösen des medizinisches Implantats(105), insbesondere nach einem der vorhergehenden Ansprüche, umfassend einen Klemmkörper (10) zum Klemmen des Implantats in der Einführvorrichtung (110) mit einem proximalen Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, wobei der Klemmkörper (10) das Implantat (105) im Klemmzustand an zumindest einer Stelle (106) kappenartig umschließt.

10. Einführvorrichtung nach Anspruch 9, wobei ein Stopper (125) vorgesehen ist, der eine Bewegung des Implantats (105) in Richtung eines proximalen Endes (115) der Einführvorrichtung (110) limitiert, wobei der Klemmkörper (10) an dem Stopper (125) anliegt und/oder der Klemmkörper (10) mit dem Stopper (125) einstückig ausgeführt ist.

11. Einführvorrichtung nach Anspruch 9 oder 10, wobei das Implantat (105) ein selbstexpandierendes Implantat (105) und/oder befestigungselementlos ausgebildet ist.

12. Verfahren zur Herstellung eines Klemmkörpers (10) einer Freigabevorrichtung (100), insbesondere nach einem der Ansprüche 1 bis 8, zum Klemmen eines medizinischen Implantats (105) in einer Einführvorrichtung (110), insbesondere nach Anspruch 9, bei welcher das Implantat (105) durch eine Relativbewegung zwischen einem ersten und einem zweiten Einführelement (52, 54) freisetzt wird, wobei der Klemmkörper (10) ein proximales Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, umfasst, aufweisend zumindest die folgenden Schritte:
- Bereitstellen eines Schlauchs (22) (Schritt I);
- Aufweiten des Schlauchs (22) an zumindest einem Ende (14) (Schritt II);
- Aufspleißen des Schlauchs (22) an einem Ende (14) in wenigstens zwei sich entlang einer Haupterstreckung (24) des Schlauchs (22) erstreckende Hüllteile (16, 18) (Schritt IV).

13. Verfahren zur Herstellung eines Klemmkörpers (10) nach Anspruch 12, wobei ein Material des Schlauchs (22) an zumindest einer Stelle (26) reduziert wird (Schritt III), um eine Stauchung des Materials zu ermöglichen (Schritt VII).

14. Verfahren zum Klemmen eines medizinischen Implantats (105) mittels eines Klemmkörpers (10) einer Freigabevorrichtung (100), insbesondere nach einem der Ansprüche 1 bis 8, in einer Einführvorrichtung (110), insbesondere nach Anspruch 9, bei welcher das Implantat (105) durch eine Relativbewegung zwischen einem ersten und einem zweiten Einführelement (52, 54) freisetzt wird, wobei der Klemmkörper (10) ein proximales Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, umfasst, aufweisend zumindest die folgenden Schritte:
- Einführen zumindest eines proximalen Endes (107) des Implantats (105) aus Richtung eines aufgeweiteten Ende (14) in eine Mantelfläche (28) des Klemmkörpers (10) (Schritt VI), die von zumindest zwei Hüllteilen (16, 18) des Klemmkörpers (10) gebildet wird;
- Umschließen des proximalen Endes (107) des Implantats (105) mit der Mantelfläche (28) der zumindest zwei Hüllteile (16, 18) des Klemmkörpers (10) (Schritt VII);
- Platzierung des Klemmkörpers (10) mit dem Implantat (105) in zumindest einem Einführelement (54) (Schritt VIII).

15. Verfahren zum Klemmen eines medizinischen Implantats (105) nach Anspruch 14, wobei das Implantat (105) während des Umschließens mit der Mantelfläche (28) der zumindest zwei Hüllteile (16, 18) des Klemmkörpers (10) zusammen gedrückt wird (Schritt VII).
